# EUROPÄISCHE PATENTSCHRIFT

(11) **EP 1 362 607 B1**
(45) Veröffentlichungstag und Bekanntmachung des Hinweises auf die Patenterteilung: **28.07.2004**
(21) Anmeldenummer: 02010301.6
(22) Anmeldetag: 07.05.2002
(51) Int. Cl.: A61M 5/158, A61M 39/10, A61M 39/14

(54) **Infusionskatheter zur subkutanen Verabreichung flüssiger Medikamente, insbesondere Insulin**
Infusion catheter for the subcutaneous administration of liquid drugs, particularly insulin
Cathéter de perfusion pour l'administration sous-cutanée de médicaments liquides, en particulier de l'insuline

(43) Veröffentlichungstag der Anmeldung: 19.11.2003
(73) Patentinhaber: Clinico GmbH, 36251 Bad Hersfeld (DE)
(72) Erfinder: Heinzerling, Jörg, 36251 Bad Hersfeld (DE)
(74) Vertreter: von Raffay, Vincenz, Dipl.-Ing.

(56) Entgegenhaltungen:
- EP-A- 1 190 673
- WO-A-01/52617
- DE-U- 29 503 099
- DE-U- 29 905 066
- US-A- 5 545 143
- US-A- 5 545 152

## Beschreibung

Die Erfindung betrifft einen Infusionskatheter nach dem Oberbegriff des Anspruches 1, wie z.B. aus EP-A-1190673 bekannt.

Derartige Infusionskatheter dienen der Verabreichung flüssiger Medikamente, beispielsweise Insulin, Schmerzmittel und anderer wiederholt zu verabreichender Medikamente. Der Katheter ist über einen Schlauch mit der Medikamentenpumpe verbunden. Es muß eine Kupplung vorgesehen sein, die eine Trennung von Katheter und Pumpe ermöglicht, damit sich der Patient entsprechend freier bewegen kann.

Es sind die unterschiedlichsten Ausgestaltungen für diese Katheter und Kupplungen bekannt. Häufig sind die Kupplungen als getrennte Elemente ausgebildet, die nicht unmittelbar Teil des Katheters sind. In dem Katheter und/oder der Kupplung befinden sich Stahlkanülen, die stark beansprucht werden und bestimmte Voraussetzungen erfüllen müssen. Zum einen dienen sie der Punktion des Patienten. Zum anderen sind sie Teil der Kupplung.

Der Erfindung liegt die Aufgabe zugrunde, ein Infusionskatheter der eingangs genannten Art zu schaffen, der gleichzeitig Kupplungselement zur Verbindung mit dem zur Medikamentenpumpe führenden Schlauch ist und der für eine sichere und dichte Verbindung auch bei wiederholtem Ver- und Entriegeln sorgt. Gleichzeitig soll der Infusionskatheter entsprechend kompakt gestaltet und sicher zu betätigen sein.

Diese Aufgabe wird grundsätzlich durch das Kennzeichen des Anspruches 1 gelöst.

Erfindungsgemäß bilden Katheter und Kupplung eine Baueinheit, so daß für eine kompakte Bauweise gesorgt ist. Die der Punktion des Patienten dienende Stahlkanüle ist Teil des ersten Kupplungselementes. Auf diese Kanüle ist das zweite Kupplungselement aufschiebbar, so daß die Verbindung mit dem Schlauch sicher hergestellt wird. Die perforierte Dichtung, die Teil des zweiten Kupplungselementes ist, wird hierbei axial auf das freie rückwärtige Ende der Kanüle aufgeschoben. Es wird für eine sichere und saubere Verbindung beim Eintauchen der Kanüle in die Dichtung gesorgt.

In vorteilhafterweise ist der Infusionskatheter so ausgebildet wie in Anspruch 2 unter Schutz gestellt. Die Griffplatte, die Teil des zweiten Kupplungselementes ist, liegt so mittig und symmetrisch, daß in ihrer Griffebene auch die Mittellinie der Kanüle liegt. Hierdurch werden beim Einstich nur Kräfte in Richtung der Mittellinie der Kanüle ausgeübt, so daß keine Momente auftreten. Die auf die Griffplatte ausgeübte Kraft beim Einstechen wird ohne Versatz auf die der Punktion dienende Kanüle übertragen.

Weiterhin kann die Infusionspumpe so ausgebildet sein, wie in Anspruch 3 unter Schutz gestellt. Bei dieser Ausgestaltung werden die beiden Kupplungselemente nach dem Verbinden durch die Bewegung in Achsrichtung der Kanüle durch Drehung miteinander verriegelt. Diese Verriegelung kann durch eine Drehung in die entgegengesetzte Richtung wieder gelöst werden. Die Drehung ist durch besondere bauliche Maßnahmen nur dann möglich, wenn die Verriegelungsstellung vollständig eingenommen wird, so daß immer sichergestellt ist, daß die Öffnung des rückwärtigen Endes der Kanüle hinter der Dichtung liegt. Hierdurch wird der ungehinderte Fluß des Medikamentes sichergestellt.

Weitere vorteilhafte Ausgestaltungen sind Gegenstand der Ansprüche 4 bis 6.

Die Ausgestaltung nach Anspruch 5 mit einer rechtwinkligen Kanüle, von denen das eine Ende der Punktion des Patienten dient und der andere um 90° abgewinkelte in die Dichtung eintaucht, stellt eine besonders vorteilhafte Ausgestaltung dar, da die Verschiebebewegung zur Verbindung der beiden Kupplungselemente dann entsprechend parallel zur Hautoberfläche des Patienten erfolgt. Die Drehbewegung zur Verriegelung der beiden Kupplungselemente erfolgt dann um das abgewinkelte rückwärtige Ende der Kanüle.

Bei Ausgestaltung nach Anspruch 7 ist eine Schutzkappe vorgesehen, die dann eingesetzt werden kann, wenn die Kupplungselemente getrennt werden, um dem Patienten eine größere Bewegungsfreiheit zu ermöglichen. Die Schutzkappe, die entsprechend wie das zweite Kupplungselement bewegbar und drehbar ist, um die gewünschten Stellung einzunehmen, schützt dann sicher das freie rückwärtige Ende der Kanüle.

Im folgenden wird die Erfindung unter Hinweis auf die Zeichnung anhand eines Ausführungsbeispieles näher erläutert.

Es zeigt:
- Fig. 1: eine geschnittene Seitenansicht einer Ausführungsform eines Infusionskatheters nach der Erfindung;
- Fig. 2: einen Schnitt gemäß der Linie A - A der Fig. 1;
- Fig. 3: einen Schnitt gemäß der Linie B - B der Fig. 1;
- Fig. 4: eine Draufsicht auf den Infusionskatheter der Fig. 1 in der Stellung, die er einnimmt, wenn er dauerhaft mit der Hautoberfläche des Patienten verbunden ist und sich in der Verriegelungsstellung befindet;
- Fig. 5: eine Seitenansicht der Darstellung der Fig. 4; und
- Fig. 6: eine geschnittene Seitenansicht, die derjenigen der Fig. 1 entspricht, wobei jedoch das zweite Kupplungselement durch eine Schutzkappe ersetzt wurde.

Der in der Zeichnung dargestellte Infusionskatheter besteht aus zwei Hauptteilen, einem ersten Kupplungselement, das mit der Hautoberfläche des Patienten zu verbinden ist, und einem zweiten Kupplungselement, der über einen Schlauch mit der nicht gezeigten Medikamentenpumpe verbunden ist.

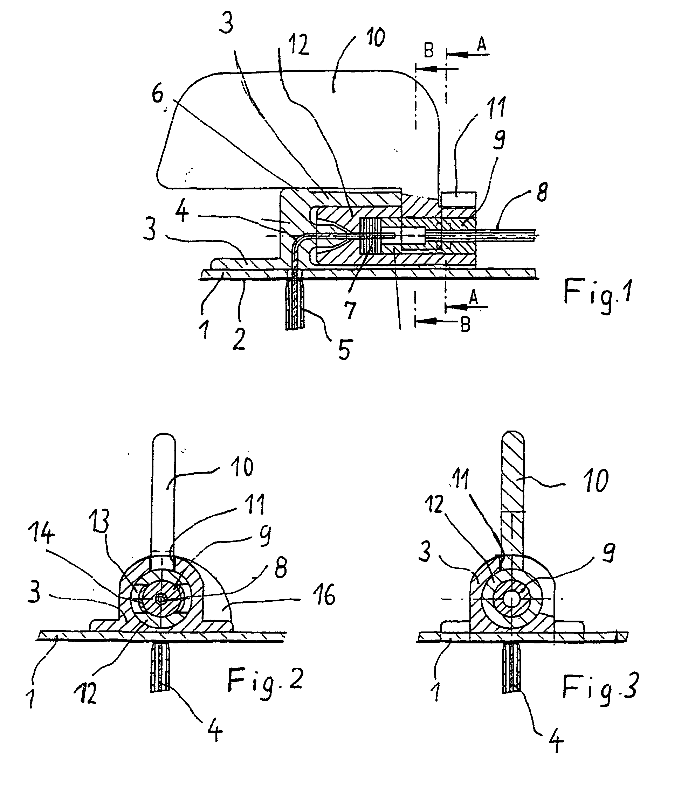

Das erste Kupplungselement wird durch einen zylindrischen Kanülenhalter 3 gebildet, der auf einer Klebeplatte 1 sitzt, deren Klebefläche durch eine Schutzfolie 2 abgedeckt ist. In diesem Kanülenhalter 3 befindet sich eine Stahlkanüle 4, die ungefähr rechtwinklig verläuft, wie es der Fig. 1 zu entnehmen ist. Das freie der Punktion des Patienten dienende Ende der Kanüle 4 ist durch ein Schutzröhrchen 5 geschützt.

Das zweite Kupplungsteil wird durch eine Hülse 12 gebildet, an der einstückig eine relativ große Griffplatte 10 ausgebildet ist. In dem Hohlraum der Hülse 12 befindet sich eine vorperforierte Dichtung oder Dichtscheibe 7, die auf der freien Stirnseite durch die Schlauchumspritzung 9 für den Schlauch 8 gehalten wird. Auf der Rückseite der zylindrischen Hülse 12 befindet sich oben ein Schlitz 11, durch den die Griffplatte 10 paßt.

In der Hülse 12 sind Hinterschnitte 13 zur Aufnahme von Vorsprüngen 14 an der Schlauchumspritzung 9 vorgesehen, damit eine dauerhafte Verbindung zwischen der Schlauchumspritzung 9 und der Hülse 12 hergestellt werden kann und die Dichtung 7 sicher an ihrem Platz in dem Hohlraum der Hülse 12 gehalten wird.

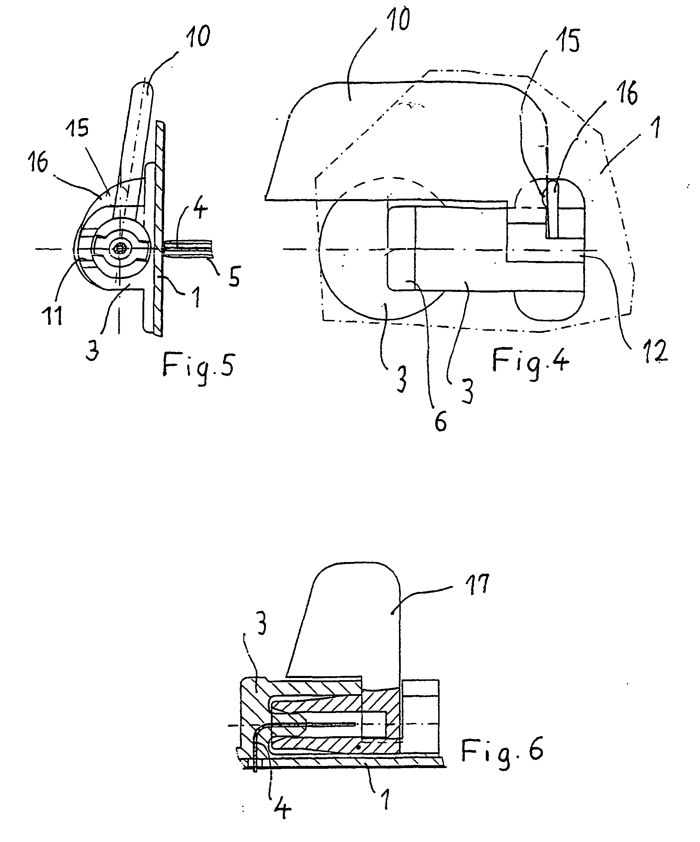

An der Hülse 12 befindet sich, wie in den Figuren 4 und 5 erkennbar, eine Platte 16, die eine Arretierung 15 für die Griffplatte 10 trägt.

In Fig. 6 ist eine Schutzkappe 17 dargestellt, die anstelle des zweiten Kupplungselementes in die Hülse des ersten Kupplungselementes eingeschoben werden kann.

Der Infusionskatheter nach der Erfindung, der gleichzeitig Kupplung zur Verbindung mit dem zur Medikamentenpumpe führenden Schlauch 8 ist, dient der Applikation an der Hautoberfläche des Patienten und ermöglicht eine Unterbrechung der Verbindung mit der Pumpe, d.h. der Schlauch kann gelöst werden.

Die beiden Kupplungselemente, die vorstehend im einzelnen beschrieben wurden, werden dadurch miteinander verbunden, daß das zweite Kupplungselement im wesentlichen gebildet durch die Hülse 12 und die Griffplatte 10 in den Hohlraum des ersten Kupplungselementes im wesentlichen gebildet durch den Kanülenhalter 3 in Achsrichtung des rückwärtigen freien Endes der Kanüle 7 eingeschoben wird. Hierbei bewegt sich die Griffplatte 10 durch den Schlitz 11. Gleichzeitig taucht das rückwärtige Ende der Kanüle 4 in die vorperforierte Dichtung 7 ein, wobei sich die Öffnung dieses rückwärtigen Endes der Kanüle 4 hinter die Dichtung 7 bewegt, wie es in Fig. 1 deutlich erkennbar ist. Diese Stellung ist dadurch festgelegt und stabil, daß auf der Hülse 12 ein Stützbereich 6 zur Anlage für die entsprechende angrenzende Fläche der Griffplatte 10 vorgesehen ist. Wenn sich der Infusionskatheter in dieser in Fig. 1 dargestellten Stellung befindet, kann dis Medikamentenpumpe kurszeitig in Betrieb gesetzt werden, um sicherzustellen, daß ein freier Medikamentenfluß stattfindet. Dann wird das Schutzröhrchen 5 entfernt und der Infusionskatheter wird in die Haut des Patienten appliziert. Anschließend wird die Schutzfolie 2 zur Freilegung der Klebefläche der Klebeplatte entfernt. Hierbei hält der Patient den Katheter an der Griffplatte 10 fest, der sich in der Ebene befindet, die durch die Kanüle 4 definiert ist. Die Druckkraft, die der Patient ausführt, erfolgt dann genau in Richtung des punktierenden Endes der Kanüle 4. Kräfte, die ein unerwünschtes Moment auf dieses Ende der Kanüle ausüben, sind nicht vorhanden.

Wenn der Infusionskatheter seine endgültige Stellung nach der Punktion einnimmt, wird die Griffplatte um ca. 90° geschwenkt, so daß sie die in Figuren 4 und 5 gezeigte Stellung einnimmt. Hierbei behindert der Schlitz 11 in dem Infusionshalter 3 diese Schwenkbewegung nicht, da er entsprechend kurz ausgebildet ist. Die Schwenkbewegung kann ungehindert durchgeführt werden. In den Figuren 4 und 5 gezeigten Stellungen wird die Griffplatte 10 durch die Arretierung 15 an der Platte 16 sicher gehalten.

Wenn die Verbindung wieder gelöst werden soll, d.h. wenn die Kupplung entriegelt werden soll, aber das die Kanüle tragende Kupplungselement am Patienten verbleiben soll, wird die Schwenk- oder Drehbewegung der Griffplatte um 90° entsprechend zurück durchgeführt, so daß die Stellung nach den Figuren 1 bis 3 wieder eingenommen wird. In dieser Stellung kann nach vorherigem Abschalten der Medikamentenpumpe das zweite durch die Hülse 12 und Griffplatte 10 gebildete Kupplungselement in Achsrichtung des rückwärtigen Endes der Kanüle 4 herausgezogen werden, wobei die Griffplatte 10 durch den Schlitz 11 gleitet. Anschließend wird die in Fig. 6 gezeigte Schutzkappe 17 entsprechend eingesetzt und das rückwärtige freie Ende der Kanüle 4 ist geschützt.

## Patentansprüche

1. Infusionskatheter zur subkutanen Verabreichung flüssiger Medikamente, insbesondere Insulin mit zwei Kupplungselementen, von denen das erste die in dem Patienten zu applizierende Kanüle und das zweite den mit der Medikamentenpumpe verbundenen Schlauch trägt, **dadurch gekennzeichnet, daß** die Kanüle (4) mit ihrem nicht der Punktion des Patienten dienenden rückwärtigen Ende unmittelbar mit dem zweiten Kupplungselement (12) verbindbar ist, daß dieses zweite Kupplungselement eine vorperforierte Dichtung (7) aufweist, die bei Verbindung der beiden Kupplungselemente (3,12) axial auf das rückwärtige Ende der Kanüle (4) aufgeschoben ist, und **daß** an dem zweiten Kupplungselement (12) eine als Einstichgriff für die Punktion der Kanüle (4) dienende Griffplatte (10) vorgesehen ist, in deren Griffebene die Mittellinie der Kanüle (4) in der Verbindungsstellung der Kupplungselemente (3,12) liegt.

2. Infusionskatheter nach Aspruch 1, **dadurch gekennzeichnet, daß** die beiden Kupplungselemente (3,12) nach dem Verbinden durch die Bewegung in Achsrichtung der Kanüle (4) durch eine Drehbewegung des zweiten Kupplungselementes (12) um die Achse der Kanüle (4) miteinander ver- und entriegelbar sind, wobei die Drehung in die Verriegelungsstellung nur bei vollständig durchgeführer Axialbewegung möglich ist.

3. Infusionskatheter nach Anspruch 1 oder 2, **dadurch gekennzeichnet, daß** die Kanüle (4) in dem ersten Kupplungselement (3) um ca. 90° umgebogen ist, so daß das rückwärtige Ende in Gebrauchsstellung parallel zu der Hautoberfläche des Patienten verläuft,
daß das zweite Kupplungselement (12) mit der Dichtung (7) entsprechend parallel zu der Hautoberfläche in das erste Kupplungselement (3) einschiebbar ist, und
daß die Griffplatte (10) beim und nach Beendigung des Einschiebens in der Ebene des Endes der Kanüle (4) liegt, die der Punktion des Patienten dient und nach vollständigen Einschieben um ca. 90° schwenkbar ist, so daß die Ebene der Griffplatte ungefähr parallel zur Hautoberfläche des Patienten liegt.

4. Infusionskatheter nach einem oder mehreren der vorstehenden Ansprüche 1 bis 3, **dadurch gekennzeichnet, daß** das erste, den Kanülenhalter (3) bildende Kupplungselement als Hohlzylinder ausgebildet ist, in dessen Hohlraum das rückwärtige Ende der Kanüle (4) liegt und in dem das als die Dichtung (7) tragende Hülse (12) mit Griffplatte (10) ausgebildete zweite Kupplungselement axial zu dem rückwärtigen Ende der Kanüle (4) einschiebbar und nach der Einnahme der Verriegelungsstellung, in der die Öffnung des rückwärtigen Endes der Kanüle (4) hinter der Dichtung (7) liegt, zum ver- und entriegeln, um die Achse des rückwärtigen Endes der Kanüle (4) drehbar ist.

5. Infusionskatheter nach Anspruch 4, **dadurch gekennzeichnet, daß** im ersten Kupplungselement (3) ein Schlitz (11) parallel zu der Achse des rückwärtigen Endes der Kanüle (4) vorgesehen ist, durch den die Griffplatte (10) zur Verbindung mit dem zweiten Kupplungselement (12) schiebbar ist und der eine Drehung des zweiten Kupplungselementes (12) verhindert, solange die vollständige Verbindung der beiden Kupplungselemente nicht hergestellt ist.

6. Infusionskatheter nach einem oder mehreren der vorstehenden Ansprüche, **gekennzeichnet durch** eine Schutzkappe (17), die anstelle des zweiten Kupplungselementes (12) zum Schutz des rückwärtigen Endes der Kanüle (4) in das erste Kupplungselement einsteckbar ist.

## Claims

1. Infusion catheter for the subcutaneous administration of liquid medicaments, particularly insulin, having two coupling elements, whereof the first carries the cannula to be applied to the patient and the second the tube connected to the medicament pump, **characterized in that** the rear end of the cannula (4) not used for the cannulization of the patient can be directly connected to the second coupling element (12), that said second coupling element has a preperforated seal (7), which on connecting together the two coupling elements (3, 12) can be axially engaged on the rear end of the cannula (4) and that on the second coupling element (12) is provided a gripping plate (10) serving as a puncture grip for cannulizing by the cannula (4) and in whose gripping plane is located the axis of the cannula (4) when the coupling elements (3, 12) are connected.

2. Infusion catheter according to claim 1, **characterized in that** after connection through the axial movement of the cannula (4), the two coupling elements (3, 12) can be locked together and unlocked by a rotary movement of the second coupling element (12) about the axis of the cannula (4), rotation in the locking position only being possible when the axial movement has been completed.

3. Infusion catheter according to claim 1 or 2, **characterized in that**, in the first coupling element (3), the cannula (4) is bent round by approximately 90°, so that the rear end is in the use position parallel to the skin surface of the patient, that the second coupling element (12) with the seal (7) can be inserted in the first coupling element (3) parallel to the skin surface and that the gripping plate (10) during and after the end of insertion is located in the plane of the end of the cannula (4) used for the cannulization of the patient and following complete insertion can be pivoted by approximately 90°, so that the gripping plate plane is approximately parallel to the skin surface of the patient.

4. Infusion catheter according to one or more of the claims 1 to 3, **characterized in that** the first coupling element forming the cannula holder (3) is constructed as a hollow cylinder, in whose cavity is located the rear end of the cannula (4) and in which the second coupling element constructed as the seal (7)-carrying sleeve (12) with the gripping plate (10) can be introduced axially to the rear end of the cannula (4) and after assuming the locking position, where the opening of the rear end of the cannula (4) is behind the seal (7), is rotatable about the axis of the rear end of the cannula (4) for locking and unlocking.

5. Infusion catheter according to claim 4, **characterized in that** the first coupling element (3) has a slot (11) parallel to the axis of the rear end of the cannula (4) through which can be slid the gripping plate (10) for connection to the second coupling element (12) and which prevents a rotation of the second coupling element (12) for as long as no complete connection has taken place between the two coupling elements.

6. Infusion catheter according to one or more of the preceding claims, **characterized by** a protective cap (17) engageable in the first coupling element in place of the second coupling element (12) for protecting the rear end of the cannula (4).

## Revendications

1. Cathéter de perfusion pour l'administration sous-cutanée de médicaments liquides, en particulier de l'insuline, muni de deux éléments d'accouplement parmi lesquels le premier porte la canule devant être implantée dans le patient, et le second porte le flexible raccordé à la pompe à médicaments, **caractérisé par le fait que** la canule (4) peut être directement reliée au second élément d'accouplement (12) par son extrémité postérieure non destinée à pénétrer dans le patient ;
**par le fait que** ce second élément d'accouplement présente une garniture d'étanchéité (7) à perforation préalable, enfilée axialement sur l'extrémité postérieure de la canule (4) lorsqu'une liaison est établie entre les deux éléments d'accouplement (3, 12) ; et
**par le fait qu'**une plaquette de préhension (10), servant de poignée d'enfoncement pour l'enfichage de la canule (4), est prévue sur le second élément d'accouplement (12), l'axe médian de ladite canule (4) étant situé dans le plan de préhension de ladite plaquette dans la position de liaison desdits éléments d'accouplement (3, 12).

2. Cathéter de perfusion selon la revendication 1,
**caractérisé par le fait que**, à l'issue de la liaison établie par le mouvement dans la direction axiale de la canule (4), les deux éléments d'accouplement (3, 12) peuvent être verrouillés l'un avec l'autre et déverrouillés l'un de l'autre par un mouvement rotatoire du second élément d'accouplement (12) autour de l'axe de ladite canule (4), sachant que la rotation, vers la position verrouillée, n'est possible que lors d'une exécution complète du mouvement axial.

3. Cathéter de perfusion selon la revendication 1 ou 2, **caractérisé par le fait que** la canule (4) est coudée d'environ 90° dans le premier élément d'accouplement (3), de sorte que l'extrémité postérieure s'étend parallèlement à la surface de la peau du patient en position d'utilisation ;
**par le fait que** le second élément d'accouplement (12) peut être inséré dans le premier élément d'accouplement (3), avec la garniture d'étanchéité (7), de manière conséquemment parallèle à la surface de la peau ; et
**par le fait que**, lors de l'insertion et à l'achèvement de celle-ci, la plaquette de préhension (10) se trouve dans le plan de l'extrémité de la canule (4), servant à ponctionner le patient et pouvant pivoter d'environ 90° après insertion complète, de sorte que le plan de ladite plaquette de préhension est à peu près parallèle à la surface de la peau du patient.

4. Cathéter de perfusion selon l'une ou plusieurs des revendications précédentes 1 à 3, **caractérisé par le fait que** le premier élément d'accouplement, matérialisant le porte-canule (3), est réalisé sous la forme d'un cylindre creux dans la cavité duquel est logée l'extrémité postérieure de la canule (4) et dans lequel le second élément d'accouplement, réalisé sous la forme d'une douille (12) portant la garniture d'étanchéité (7) et associé à la plaquette de préhension (10), peut être inséré axialement par rapport à l'extrémité postérieure de la canule (4), et peut tourner autour de l'axe de l'extrémité postérieure de la canule (4), en vue du verrouillage et du déverrouillage, après qu'a été prise la position verrouillée dans laquelle l'ouverture de l'extrémité postérieure de la canule (4) est située derrière la garniture d'étanchéité (7).

5. Cathéter de perfusion selon la revendication 4, **caractérisé par le fait qu'**une fente (11) est prévue dans le premier élément d'accouplement (3), parallèlement à l'axe de l'extrémité postérieure de la canule (4), ladite fente autorisant le coulissement de la plaquette de préhension (10), en vue de la liaison avec le second élément d'accouplement (12), et interdisant une rotation dudit second élément d'accouplement (12) tant que n'est pas instaurée la solidarisation complète des deux éléments d'accouplement.

6. Cathéter de perfusion selon l'une ou plusieurs des revendications précédentes, **caractérisé par** un capuchon protecteur (17) pouvant être emboîté dans le premier élément d'accouplement, en substitution du second élément d'accouplement (12), en vue de protéger l'extrémité postérieure de la canule (4).
